(19) Europäisches Patentamt
European Patent Office
Office européen des brevets

(11) Veröffentlichungsnummer: **0 224 771 A1**

(12) **EUROPÄISCHE PATENTANMELDUNG**

(21) Anmeldenummer: 86115785.7

(22) Anmeldetag: 13.11.86

(51) Int.Cl.[4]: **A 01 N 47/36**
**C 07 D 333/38**

(30) Priorität: 26.11.85 DE 3541629

(43) Veröffentlichungstag der Anmeldung:
10.06.87 Patentblatt 87/24

(84) Benannte Vertragsstaaten:
DE FR IT

(71) Anmelder: BAYER AG
Konzernverwaltung RP Patentabteilung
D-5090 Leverkusen 1 Bayerwerk(DE)

(72) Erfinder: Hallenbach, Werner, Dr.
Kleiststrasse 10
D-4018 Langenfeld(DE)

(72) Erfinder: Lindel, Hans, Dr.
Carl-Duisberg-Strasse 321
D-5090 Leverkusen 1(DE)

(72) Erfinder: Brandes, Wilhelm, Dr.
Eichendorffstrasse 3
D-5653 Leichlingen(DE)

(54) **Selektiv-fungizide Verwendung von 1-(3-Ethoxycarbonyl-4-ethyl-5-methyl-2-thienyl)-3-methylharnstoff.**

(57) Selektiv-fungizide Verwendung des 1-(3-Ethoxycarbonyl-4-ethyl-5-methyl-2-thienyl)-3-methylharnstoff der Formel (I)

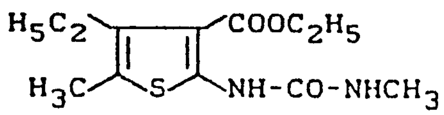

zur Bekämpfung von Botrytispilzen.

EP 0 224 771 A1

0224771

BAYER AKTIENGESELLSCHAFT
Konzernverwaltung RP
Patentabteilung

5090 Leverkusen, Bayerwerk
25. Nov. 1985
Bas/Kü-c
IIb

# Selektiv-fungizide Verwendung von 1-(3-Ethoxycarbonyl-4-ethyl-5-methyl-2-thienyl)-3-methylharnstoff

Die vorliegende Erfindung betrifft die Verwendung von 1-(3-Ethoxycarbonyl-4-ethyl-5-methyl-2-thienyl)-3-methylharnstoff als selektives Fungizid gegen Botrytispilze.

Es ist bereits bekannt geworden, daß bestimmte Thienylharnstoffe, wie z. B. 1-(4,5-Dimethyl-3-ethoxycarbonyl-2-thienyl)-3-methylharnstoff, eine gute fungizide Wirksamkeit besitzen (vergl. z. B. US-PS 3 823 161). Über die vom wirtschaftlichen Standpunkt aus hoch interessante Wirksamkeit gegen Botrytispilze ist jedoch nichts bekannt.

Es wurde nun gefunden, daß speziell die Verbindung der Formel (I)

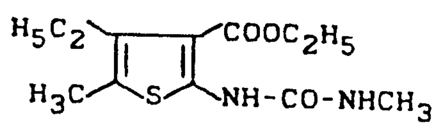

(I)

eine besonders gute selektive fungizide Wirksamkeit gegen Botrytispilze besitzt.

Überraschenderweise zeigt die Verbindung der Formel (I) eine erheblich höhere selektive fungizide Wirkung als die aus dem Stand der Technik bekannte Verbindung 1-(4,5-Dimethyl-3-ethoxycarbonyl-2-thienyl)-3-methylharnstoff (vergl. z. B. US-PS 3 823 161).

Der erfindungsgemäß zu verwendende Wirkstoff der Formel (I) läßt sich nach bekannten Methoden herstellen (vergl. z. B. EP-OS 4 931, DE-AS 20 40 579, US-PS 3 823 161, DE-AS 26 27 935 und eine eigene deutsche nicht vorveröffentlichte Anmeldung P 3 529 247.4 vom 16.08.1985). So kann man z. B. 2-Amino-3-ethoxycarbonyl-4-ethyl-5-methyl-thiophen mit Methylisocyanat in Gegenwart von inerten Verdünnungsmitteln und Katalysatoren, wie z. B. Pyridin, bei Temperaturen zwischen 20 °C und 70 °C gemäß dem folgenden Formelschema umsetzen:

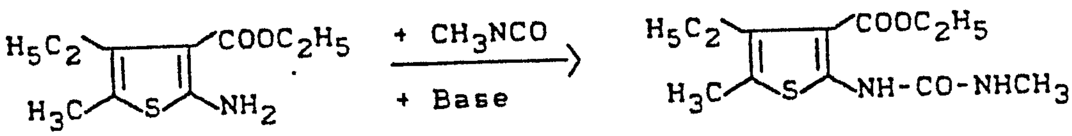


Der erfindungsgemäß einzusetzende Wirkstoff weist eine starke selektive fungizide Wirkung auf und kann zur Bekämpfung von unerwünschten Mikroorganismen praktisch eingesetzt werden. Der Wirkstoff ist für den Gebrauch als selektives Fungizid geeignet.

Eine besonders gute Wirksamkeit besitzt der erfindungsgemäß einzusetzende Wirkstoff gegen Botrytispilze, z. B.

gegen Botrytis cinerea, den Erreger des Grauschimmels an Gartenbohnen, Salat, Erdbeeren und Reben. Vorzugsweise wird der Wirkstoff an Reben, Erdbeeren und Salat eingesetzt.

Der Wirkstoff kann in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen des Wirkstoffs mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine,

und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgemäße Wirkstoff kann in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Der Wirkstoff kann als solcher, in Form seiner Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

0224771

Herstellungsbeispiel

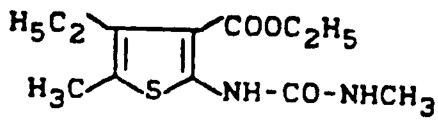

Zu 7 g (0,033 Mol) 2-Amino-3-ethoxycarbonyl-4-ethyl-5-methylthiophen in 30 ml trockenem Pyridin werden 3,75 g (0,066 Mol) Methylisocyanat gegeben und dann 3 Stunden bei 60° C gerührt. Nach Abkühlung wird in 1 l verdünnte Salzsäure eingerührt. Der Niederschlag wird abgesaugt und getrocknet.

Man erhält 5,6 g (63 % der Theorie) 1-(3-Ethoxycarbonyl-4-ethyl-5-methyl-2-thienyl)-3-methyl-harnstoff vom Schmelzpunkt 137° C.

Beispiel A

Botrytis-Test (Bohne)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Der 1-(3-Ethoxycarbonyl-4-ethyl-5-methyl-2-thienyl)-3-methyl-harnstoff zeigt eine deutliche Überlegenheit in der Wirksamkeit gegenüber dem Stand der Technik (A).

## Tabelle A

Botrytis-Test (Bohne)/protektiv

| Wirkstoff | Befall in % bei einer Wirkstoffkonzentration von 100 ppm |
|---|---|
| 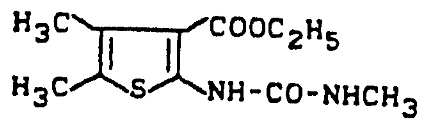<br>(bekannt) (A) | 29 |
| 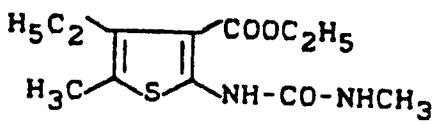 | 4 |

Beispiel B

Botrytis-Test (Rebe)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Der 1-(3-Ethoxycarbonyl-4-ethyl-5-methyl-2-thienyl)-3-methyl-harnstoff zeigt eine deutliche Überlegenheit in der Wirksamkeit gegenüber dem Stand der Technik (A).

Tabelle B

Botrytis-Test (Rebe)/protektiv

| Wirkstoff | Befall in % bei einer Wirkstoffkonzentration von 25 ppm |
|---|---|
| 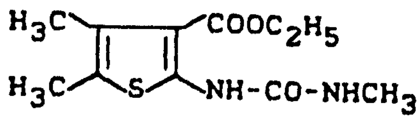 (bekannt) (A) | 33 |
| 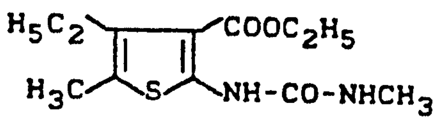 | 20 |

Patentansprüche

1. Verwendung von 1-(3-Ethoxycarbonyl-4-ethyl-5-methyl-2-thienyl)-3-methylharnstoff der Formel (I)

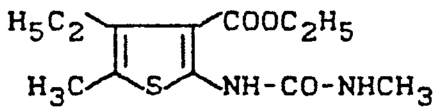

(I)

als selektives Fungizid gegen Botrytispilze.

2. Verfahren zur Bekämpfung von Botrytispilzen, dadurch gekennzeichnet, daß man die Verbindung der Formel (I) gemäß Anspruch 1 auf Botrytispilze und/oder ihren Lebensraum einwirken läßt.

3. Verwendung von 1-(3-Ethoxycarbonyl-4-ethyl-5-methyl-2-thienyl)-3-methylharnstoff gemäß Anspruch 1, in Anwendungskonzentrationen im Bereich von 0,0001 bis 1 Gewichtsprozenten.

Europäisches Patentamt

# EUROPÄISCHER RECHERCHENBERICHT

0224771
Nummer der Anmeldung

EP 86 11 5785

**EINSCHLÄGIGE DOKUMENTE**

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| D,X | DE-A-2 122 636 (ESSO RESEARCH AND ENGINEERING) * Seite 3, Verbindung 10; Seite 10, Zeilen 8-18, Beispiele 27-29; Patentansprüche 1,26-28 * --- | 1-3 | A 01 N 47/36<br>C 07 D 333/38 |
| D,A | EP-A-0 004 931 (BAYER) * Seite 24, Zeilen 28-30; Patentansprüche * --- | 1-3 | |
| D,A | DE-A-2 040 579 (MAY & BAKER) * Seite 16, Zeilen 13-17; Patentansprüche * --- | 1-3 | |
| A | CHEMICAL ABSTRACTS, Band 92, Nr. 9, 3. März 1980, Seite 650, Zusammenfassung Nr. 76205r, Columbus, Ohio, US; J.V. RAM: "Thiophenes, pyrazolothiophenes and pyrimidothiophenes as pesticides", & ARCH. PHARM. (Weinheim, Ger.) 1979, 312(9), 726-33 * Insgesamt * --- -/- | 1-3 | **RECHERCHIERTE SACHGEBIETE (Int. Cl.4)**<br>A 01 N |

Der vorliegende Recherchenbericht wurde für alle Patentansprüche erstellt.

| Recherchenort | Abschlußdatum der Recherche | Prüfer |
|---|---|---|
| DEN HAAG | 11-03-1987 | FLETCHER A.S. |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82

Europäisches Patentamt

**0224771**

# EUROPÄISCHER RECHERCHENBERICHT

Nummer der Anmeldung

EP 86 11 5785

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | JOURNAL OF PHARMACEUTICAL SCIENCES, Band 65, Nr. 5, Mai 1976, Seiten 660-664, Washington, D.C., US; M.B. DEVANI et al.: "Synthesis of 3-substituted thieno[2,3-d]pyrimidin-4(3H)-one-2-mercaptoacetic acids and their ethyl esters for pharmacological screening" * Insgesamt * | 1-3 | |
| | ----- | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| DEN HAAG | 11-03-1987 | FLETCHER A.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82